# EUROPEAN PATENT SPECIFICATION

(11) **EP 0 603 451 B1**
(45) Date of publication and mention of the grant of the patent: **19.03.1997**
(21) Application number: 92830682.8
(22) Date of filing: 23.12.1992
(51) Int. Cl.: A61N 1/26, A61N 1/32

(54) **A muscle electrostimulation device for passive gymnastics, in particular for facial cosmetics**
Einrichtung zur elektrischen Reizung von Muskeln bei passiver Gymnastik, insbesondere zur kosmetischen Gesichtsbehandlung
Dispositif d'électrostimulation musculaire de gymnastique passive, en particulier pour le traitement cosmétique de la face

(43) Date of publication of application: 29.06.1994
(73) Proprietor: VUPIESSE ITALIA S.A.S., di VALENTINI E PAOLIZZI E C., 47037 Rimini (RN) (IT)
(72) Inventor: Paolizzi, Marco, I-47037 Rimini (FO) (IT)
(74) Representative: Lanzoni, Luciano

(56) References cited:
- WO-A-91/07207
- FR-A- 1 121 462
- GB-A- 387 960
- US-A- 4 062 364

## Description

The invention relates to a muscle electrostimulation device for passive gymnastics, in particular for facial cosmetics.

In the field of facial cosmetics several devices are known which electrically stimulate the muscle bundles of the face, which devices, using at least one pair of electrodes placed in contact with the skin and connected to a microcurrent generator, appropriately pulsed and thus innocuous, determine an involuntary rhythmic contraction of the facial muscles (passive gymnastics) which is aimed at offering the user a recuperation of facial muscle tone and an aid to moderating facial aging lines.

A first way of performing a device of the above mentioned type, which more particularly corresponds to the preamble of claim 1, is described in the document WO-91/07207.

Such a device comprises a case equipped with a workhead equipped with electrodes and a handgrip which is arranged at an angle with respect to the workhead. More particularly, the workhead is equipped with four electrodes which are essential so that a large area complex field is created.

The electrodes, which have a metallic conducting surface, are located on the work head and project from the device case. Furthermore, they conduct the electric current through the body of the user by means of the interposition of a conducting gel which is spread on the user's skin before the electrostimulator is applied.

It is also known a document (US-A-4062364) wherein the electrodes are reciprocally aligned, transversally to a plane passing through the symmetry axis of the case handgrip, forming a right angle with respect to the said plane.

The fundamental drawback of the prior art electrostimulation devices lies in the fact that, due to the arrangement of the electrodes on the head, during use the stimulation of the facial muscles is of scarse efficiency.

Further, since the handgrip of the device is angled with respect to the head, in order to provide an ergonomic grip during use of the device in the case of the above-mentioned orthogonal situation, the result is that the electrodes become arranged, during use, in a position which is substantially horizontal to the face, and thus is arranged not entirely correspondingly with the substantially vertical development of the facial muscles.

A further drawback of the prior art electrostimulator devices lies in the fact that,in order to function, they require the use of conducting gel which is unpleasant on the skin for most users, with the removal operation also being distasteful.

The aim of the present invention, as it is characterised in the claims that follow, is thus that of obviating the above-mentioned drawbacks.

The invention solves the problem of providing a muscle electrostimulation device for passive gymnastics, in particular for facial cosmetics, comprising a support case for a workhead equipped with a handgrip which is angled with respect to the head itself and having electrodes on the said head which are aligned in such a way as to form an acute angle with respect to the halfway point plane of the handgrip determining, correspondingly, a substantially vertical location of the electrodes during the use of the device.

The head of the device and the electrodes are further conformed in such a way that the said electrodes self-serve of a conducting liquid contained in corresponding cavities made in the head itself.

The fundamental advantage of the invention consists essentially in the fact that the special arrangements of the electrodes permits a more efficient and rational stimulation of the face muscles.

A second advantage consists in the fact of not requiring the use of a conducting gel, but simply using a liquid which, apart from not being distasteful for the user and not fouling the constituent parts of the device, can be dosed strictly according to the specific needs of the user.

Further characteristics and advantages of the present invention will better emerge from the detailed description that follows, of an embodiment of the invention, herein illustrated purely in the form of a non-limiting example in the accompanying figures, in which:
- figure 1 shows the invention according to a frontal view of the whole;
- figure 2 shows the invention according to a lateral view of the whole;
- figure 3 shows a constituent particular of the invention shown respectively according to a plan view, a perspective view and a lateral view;
- figure 4 shows an electrode of the invention represented in full view but transversally sectioned;
- figure 5 shows a further embodiment of the electrode of figure 4, shown in a transversally-sectioned view of the whole.

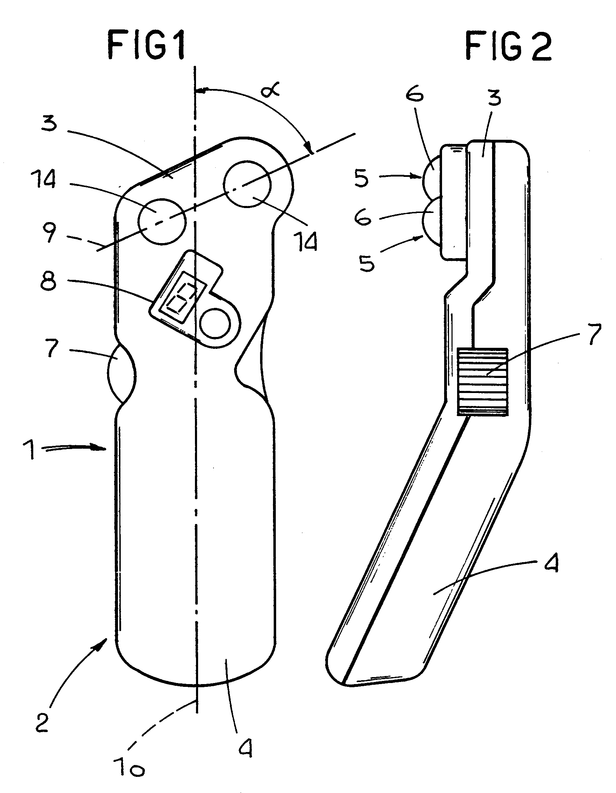

With reference to the figures, figure 1 shows a device 1 for muscular electrostimulation for passive gymnastics, in particular for facial cosmetics, comprising a case 2, preferably of plastic material, equipped with a workhead 3 and a handgrip 4 reciprocally angled one to the other.

The said angle (figure 2) has the aim of enabling the workhead 3 to be located correctly with respect to the skin during use, allowing the handgrip 4 to be gripped comfortably at the same time.

The workhead 3 supports a pair of electrodes 5 which project from the case 2 and are equipped with metallic conducting surfaces 6, which surfaces are cupola-shaped and electrically connected to a conventional microcurrent pulse generator, not shown in the figures, contained internally to the case 2 and activatable by means of an appropriate on-off and treatment time-selection button 7, the time value being visualised by a visualiser 8 situated on the case 2 of the device 1.

The electrodes 5 are located on the workhead 3 in such a way as to be arranged along a line 9 forming an acute angle (α) of about 65.5 degrees with a centre-line plane 10 of the handgrip 4 (figure 1).

The said value of angle α permits, when gripping the handgrip 4, a substantially vertical arrangement of the electrodes, corresponding to the development of the facial muscles.

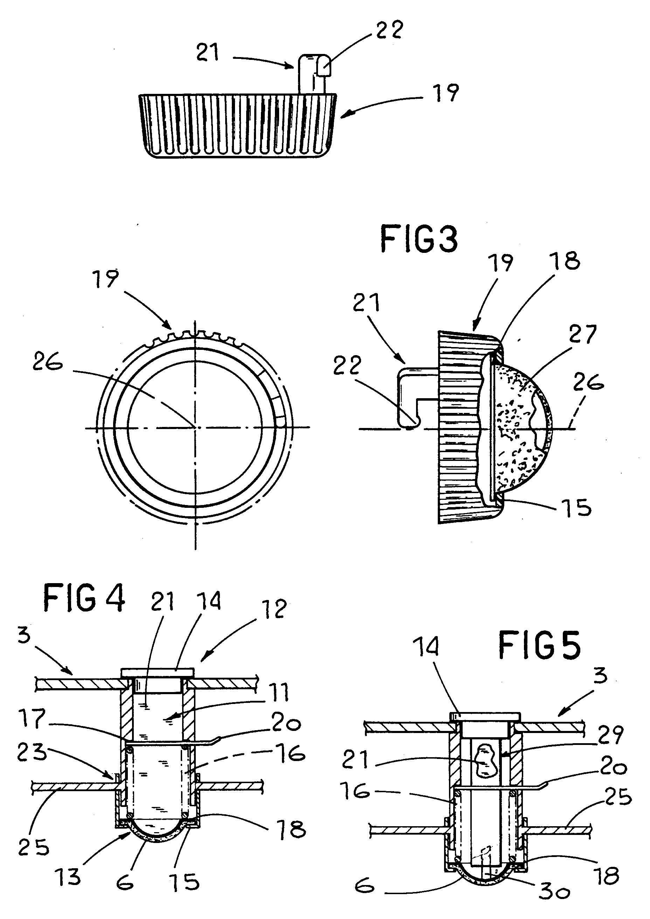

With reference to figure 4, it can be seen that the workhead 3 is crossed by tubular chambers 11 for containing a conducting liquid 21.

The said chambers 11 are closed at their opposite ends 12, 13, on one side by a moveable cap 14 for the reintegration of the liquid, and on the other side by the said conducting surface 6 of the electrodes 5. The said conducting surface 6 is interpositioned between a projection 15 of a ring-nut 19, frontally meeting a peripheral edge 18 of the conducting surface 6, and a spring 16 associated to a channel 17 of the tubular chamber 11.

The conducting surface 6 is elastically pressed against the projection 15 of the ring-nut 19, realising thus a watertight seal of the chambers 11 between the projection 15 and the peripheral edge 18 of the conducting surface 6 itself when the device 1 is in the rest condition; but instead determining the exit of the conducting liquid 21 from the chambers 11 when the device 1 is pressed against the skin of the user.

A conductor 20 of electricity, preferably having a circular crown shape, arranged crossing the chambers 11, between the channel 17 and the spring 16, allows the electrical current to reach the conducting liquid 21 which ensures the conduction of the electrical current up to the conducting surface 6 of the electrodes 5. Naturally the device 1 function correctly in the absence of the conducting liquid 21, since, in such a case, the conduction of the electrical current is effected through the spring 16, interpositioned between the conducting surface 6 and the conductor 20 itself.

With reference to figure 3 it can be observed that the ring-nut 19 is movable with respect to the workhead 3 so as to permit of easy cleaning and maintenance of the electrodes 5. For this purpose the ring-nut 19 is equipped with a shaped appendage 21, arranged projectingly, equipped with a transversal prong 22 which can be inserted into a correspondingly shaped channel 23, cut on a wall 25 of the workhead 3 so as to be stably but movably fixed there after the rotation of the said ring-nut 19 about its own symmetry axis.

Figure 3 also shows that the conducting surface 6 of the electrodes 5 is preferably covered with a layer 27 of spongey material which is humidified by the conducting liquid 21 and keeps the conducting surface 6 continually dampened during the use of the device 1.

The invention as it is conceived herein is susceptible to numerous modifications and variations, all falling within the field of the inventive idea as defined in the appended claims. Further, all the details can be substituted by technically equivalent one.

For example, the liquid 21 can be constituted by water; however it is understood that this conducting liquid 21 could advantageously be constituted by a cosmetic product packed and contained in cartridges 29 of the type with breakable membrane opening.

The said cartidges 29 could be inserted, as shown in figure 5, internally to the chambers 11 in which case the conducting surface 6 of the electrodes 5 could be made in such a way as to envisage a pointed appendage 30 turned internally to the chambers 11 so as to determine the breaking of the membrane 29 when the said membrane is pressed posteriorly by means of the cap 14 against the conducting surface itself.

## Claims

1. A muscle electrostimulation device (1) for passive gymnastics, in particular for facial cosmetics, comprising a case (2) equipped with a workhead (3) equipped with electrodes (5), and a handgrip (4) which is arranged at an angle with respect to the workhead (3), characterised in that the said electrodes (5) are two, and are positioned on the said workhead (3) in such a way as to be arranged along a line (9) which forms an acute angle alpha with a centre-line plane (10) of the said handgrip (4), and which extends through the workhead (3).

2. A device, as in claim 1, characterised in that the said angle α is of 65.5 degrees.

3. A device as in claim 1, characterised in that the said electrodes (5) exhibit cupola-conformed conducting surfaces (6).

4. A device as in claim 1, wherein the said electrodes (5) have a metallic conducting surface (6), characterised in that the said workhead (3) is equipped with tubular chambers (11) for containing a electricity-conducting liquid (21), which chambers (11) are closed at their opposite ends (12, 13) respectively by a removable cap (14) for the reinstatement of the conducting liquid and by the said conducting surface (6) of the electrodes (5); the said conducting surface (6) being interpositioned between a frontal-striking projection (15), belonging to a ring-nut (19) removably associated with the said workhead (3), and a spring (16) associated to a channel (17) of the said tubular chamber (11) in such a way as to determine, on elastic compression of the said spring (16) corresponding to the pressure of the said device (1) on a skin surface, the exit of the conducting liquid (21) from the said chamber (11).

5. A device, as in claim 1, characterised in that the said electrodes (5) are coated externally to their conducting surface (6) with a layer (27) of spongy material destined to humidify with the conducting liquid (21), keeping the said conducting surface (6) constantly humidified.

6. A device as in claim 4, characterised in that the said conducting liquid (21) is water.

7. A device as in claim 4, characterised in that the said conducting liquid (21) is a cosmetic liquid.

8. A device as in claim 4, characterised in that it comprises internally to the said tubular chamber (11), a cartridge (29) containing a cosmetic product, the said conducting surface (6) being equipped with a pointed appendage (30) upturned towards the inside of the said chamber (11) and aimed at determining the piercing of the said cartridge (29) when the said cartridge (29) is pressed against the conducting surface (6).

9. A device as in claim 4, characterised in that the said chamber (11) is crossed by an electricity conductor (20) immersed in the said liquid (21).

10. A device as in claim 4, characterised in that the said conductor (20) is interpositioned between the said channel (17) and the said spring (16).

11. A device as in claim 4, characterised in that the said ring-nut (19) is equipped with a shaped appendage (21), arranged projectingly, equipped with a transversal prong (22) engageable in a channel (23) correspondingly shaped and cut on a will (25) of the workhead (3) in such a way as to be stably but removably engaged, after a rotation of the said ring-nut (19) about its own axis of symmetry (26).

## Patentansprüche

1. Einrichtung (1) zur elektrischen Reizung von Muskeln bei passiver Gymnastik, insbesondere zur kosmetischen Gesichtsbehandlung, bestehend aus einem Gehäuse (2), das mit einem Arbeitskopf (3) versehen ist, welcher Elektroden (5) aufweist, und einem Handgriff (4), der in einem Winkel in Bezug auf dem Arbeitskopf (3) angeordnet ist, dadurch gekennzeichnet, daß die besagten Elektroden (5) zwei sind und derart auf dem besagten Arbeitskopf (3) angeordnet sind, daß sie entlang einer Linie (9) angeordnet sind, die mit einer Mittellinienebene (10) des besagten Handgriffs (4) einen spitzen Winkel Alpha bildet, und die durch den Arbeitskopf (3) verläuft.

2. Einrichtung nach Anspruch 1, dadurch gekennzeichnet, daß der besagte Winkel α 65.5 Grad beträgt.

3. Einrichtung nach Anspruch 1, dadurch gekennzeichnet, daß die besagten Elektroden (5) eine kuppelförmig ausgebildete Leiterfläche (6) aufweisen.

4. Einrichtung nach Anspruch 1, wobei die besagten Elektroden (5) eine metalleitende Fläche (6) aufweisen, dadurch gekennzeichnet, daß der besagte Arbeitskopf (3) mit rohrförmigen Kammern (11) versehen ist zur Aufnahme einer Elektrizität leitenden Flüssigkeit (21), wobei die Kammern (11) an ihren gegenüberliegenden Enden (12, 13) jeweils durch einen abnehmbaren Stopfen (14) verschlossen sind, zur Wiederherstellung der Leiterflüssigkeit, sowie durch die besagte Leiterfläche (6) der Elektroden (5); die besagte Leiterfläche (6) zwischen einem Frontal-Anschlag-Ansatz (15), der zu einer Ringmutter (19) gehört, welche lösbar mit dem besagten Arbeitskopf (3) verbunden ist, und einer Feder (16) angeordnet ist, die derart mit einer Nut (17) der besagten rohrförmigen Kammer (11) in Verbindung steht, um bei elastischer Kompression der besagten Feder (16), entsprechend dem Druck der besagten Einrichtung (1) auf einer Hautoberfläche, den Austritt der Leiterflüssigkeit (21) aus der besagten Kammer (11) festzulegen.

5. Einrichtung nach Anspruch 1, dadurch gekennzeichnet, daß die besagten Elektroden (5) extern an ihrer Leiterfläche (6) mit einer Schicht (27) aus schwammförmigen Material überzogen sind, die dazu vorgesehen ist, die Leiterflüssigkeit (21) aufzusaugen, die besagte Leiterfläche (6) konstant befeuchtet haltend.

6. Einrichtung nach Anspruch 4, dadurch gekennzeichnet, daß die besagte Leiterflüssigkeit (21) Wasser ist.

7. Einrichtung nach Anspruch 4, dadurch gekennzeichnet, daß die besagte Leiterflüssigkeit (21) eine Kosmetikflüssigkeit ist.

8. Einrichtung nach Anspruch 4, dadurch gekennzeichnet, daß sie im Inneren zu der besagten rohrförmigen Kammer (11) mit einer Patrone (29) versehen ist, die ein Kosmetikprodukt enthält, die besagte Leiterfläche (6) mit einem zugespitzten Ansatz (30) versehen ist, der zur Innenseite der besagten Kammer (11) hin nach oben gerichtet ist und geeignet ist, das Eindringen der besagten Patrone (29) zu bestimmen, wenn die besagte Patrone (29) gegen die Leiterfläche (6) gedrückt wird.

9. Einrichtung nach Anspruch 4, dadurch gekennzeichnet, daß die besagte Kammer (11) durch einen Elektrizitätsleiter (20) durchquert wird, der in die besagte Flüssigkeit (21) eingetaucht ist.

10. Einrichtung nach Anspruch 4, dadurch gekennzeichnet, daß der besagte Leiter (20) zwischen der besagten Nut (17) und der besagten Feder (16) angeordnet ist.

11. Einrichtung nach Anspruch 4, dadurch gekennzeichnet, daß die besagte Ringmutter (19) mit einem geformten Ansatz (21) versehen ist, der vorspringend angeordnet und mit einer quer verlaufenden Nase (22) versehen ist, die in eine entsprechenderweise ausgebildeten und in einer Wand (25) des Arbeitskopfes (3) eingearbeiteten Nut (23) derart einfügbar ist, daß sie nach einer Drehung der besagten Ringmutter (19) um ihre eigene Symmetrieachse (26) stabil und auf jeden Fall lösbar eingefügt werden kann.

## Revendications

1. Un dispositif pour la stimulation électrique des muscles (1) pour la gymnastique passive, en particulier pour les cosmétiques du visage, comprenant un boîtier (2) équipé d'une tête de travail (3) munie d'électrodes (5), et une poignée (4) disposée de biais par rapport à la tête de travail (3), caractérisé en ce que lesdites électrodes (5) sont au nombre de deux, et sont disposées sur ladite tête de travail (3) suivant une ligne (9) qui forme un angle aigu alpha avec une ligne médiane (10) de la poignée (4) susmentionnée, et qui s'étend d'un bout à l'autre de la tête de travail (3).

2. Un dispositif selon la revendication 1, caractérisé en ce que ledit angle α est de 65,5 degrés.

3. Un dispositif selon la revendication 1, caractérisé en ce que lesdites électrodes (5) présentent des surfaces conductrices en forme de coupole (6).

4. Un dispositif selon la revendication 1, dans lequel lesdites électrodes (5) ont une surface conductrice métallique (6), caractérisé en ce que ladite tête de travail (3) est pourvue de chambres tubulaires (11) destinées à contenir un liquide conducteur d'électricité (21), lesquelles chambres (11) sont respectivement fermées à leurs extrémités opposées (12, 13) par un capuchon amovible (14) pour la réintroduction du liquide conducteur et par ladite surface conductrice (6) des électrodes (5) ; ladite surface conductrice (6) étant interposée entre une saillie avant de butée (15), appartenant à un collier (19) couplé de façon amovible à ladite tête de travail (3), et un ressort (16) couplé à un tube (17) de ladite chambre tubulaire (11) de façon à établir, sur compression élastique du ressort (16) en question correspondant à la pression du dispositif (1) susmentionné sur la surface de la peau, la sortie du liquide conducteur (21) de ladite chambre (11).

5. Un dispositif selon la revendication 1, caractérisé en ce que lesdites électrodes (5) sont revêtues à l'extérieur de leur surface conductrice (6) d'une couche (27) de matière éponge destinée à s'humidifier au contact du liquide conducteur (21), maintenant ainsi ladite surface conductrice (6) humidifiée en permanence.

6. Un dispositif selon la revendication 4, caractérisé en ce que ledit liquide conducteur (21) est de l'eau.

7. Un dispositif selon la revendication 4, caractérisé en ce que ledit liquide conducteur (21) est un liquide cosmétique.

8. Un dispositif selon la revendication 4, caractérisé en ce qu'il comprend, à l'intérieur de ladite chambre tubulaire (11), une cartouche (29) contenant un produit cosmétique, ladite surface conductrice (6) étant munie d'un prolongement en pointe (30) orienté vers l'intérieur de ladite chambre (11) et destiné à percer ladite cartouche (29) lorsque cette même cartouche (29) est pressée contre la surface conductrice (6).

9. Un dispositif selon la revendication 4, caractérisé en ce que ladite chambre (11) est traversée par un conducteur d'électricité (20) plongé dans ledit liquide (21).

10. Un dispositif selon la revendication 4, caractérisé en ce que ledit conducteur (20) est positionné entre ledit tube (17) et ledit ressort (16).

11. Un dispositif selon la revendication 4, caractérisé en ce que ledit collier (19) est pourvu d'un prolongement profilé (21), disposé en saillie, pourvu à son tour d'une dent transversale (22) pouvant s'engager dans un tube (23) profilé de façon correspondante et coupé dans une paroi (25) de la tête de travail (3) de manière à être engagée de façon stable mais amovible, après rotation du collier (19) susmentionné sur son axe de symétrie (26).
